(19) Europäisches Patentamt
European Patent Office
Office européen des brevets

(11) **EP 3 877 017 B1**

(12) **EUROPÄISCHE PATENTSCHRIFT**

(45) Veröffentlichungstag und Bekanntmachung des Hinweises auf die Patenterteilung:
**16.08.2023   Patentblatt 2023/33**

(21) Anmeldenummer: **20820368.7**

(22) Anmeldetag: **03.12.2020**

(51) Internationale Patentklassifikation (IPC):
***A61M 1/36*** *(2006.01)*

(52) Gemeinsame Patentklassifikation (CPC):
**A61M 1/3624; A61M 1/3627;** A61M 2205/18; A61M 2205/3331; A61M 2205/3389

(86) Internationale Anmeldenummer:
**PCT/EP2020/084542**

(87) Internationale Veröffentlichungsnummer:
**WO 2021/110874 (10.06.2021 Gazette 2021/23)**

(54) **BLUTBEHANDLUNGSMASCHINE MIT AUTOMATISCHEN FÜLLSTANDSÜBERWACHUNG UND -REGELUNG EINES LUFTABSCHEIDERS MITTELS DRUCKPULSFREQUENZANALYSE**

BLOOD-TREATMENT MACHINE WITH MONITORING AND REGULATION OF AN AIR SEPARATOR BY PRESSURE-PULSE-FREQUENCE ANALYSIS

MACHINE POUR TRAITEMENT DU SANG AVEC MONITORISATION ET RÉGULATION D'UN SÉPARATEUR D'AIR PAR ANALYSE DE LA FRÉQUENCE DE PULSATIONS DE PRESSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.12.2019   DE 102019133075**

(43) Veröffentlichungstag der Anmeldung:
**15.09.2021   Patentblatt 2021/37**

(73) Patentinhaber: **B. Braun Avitum AG**
**34212 Melsungen (DE)**

(72) Erfinder: **RITTER, Kai-Uwe**
**34212 Melsungen (DE)**

(74) Vertreter: **Winter, Brandl - Partnerschaft mbB**
**Alois-Steinecker-Straße 22**
**85354 Freising (DE)**

(56) Entgegenhaltungen:
**US-A1- 2016 310 657     US-A1- 2017 333 616**

**Beschreibung**

Die vorliegende Erfindung betrifft eine Blutbehandlungsvorrichtung zur extrakorporalen Blutbehandlung, umfassend zumindest ein Blutleitungssystem und zumindest ein Kammerbehältnis zur Abscheidung von Blasen aus dem zu behandelnden Blut, aufweisend eine Blutpumpvorrichtung, die dazu ausgebildet ist, das Blut zu pumpen und Druckpulse mit einer vordefinierten Frequenz in dem Blutleitungssystem zu erzeugen. Des Weiteren betrifft die Erfindung ein Verfahren zur Überwachung des Füllstandes des Blutes in der Blutbehandlungsvorrichtung.

Aus dem Stand der Technik sind extrakorporale Blutbehandlungsmaschinen bereits seit langem bekannt. Beispielsweise offenbart die WO 93/01858 A eine Blutbehandlungsmaschine mit einer Steuereinheit, die ein Signal ausgibt, um eine venöse Schlauchklemme zu schließen, wenn Luft/Gas in einem venösen Abschnitt des extrakorporalen Blutkreislaufs erfasst wird. Die Luft wird aus dem venösen Abschnitt entfernt, indem eine sterile Spritze in eine Entlüftungsleitung eines Filters einer Luftauffangkammer eingeführt wird, ein Ventil in der Entlüftungsleitung geöffnet wird und die Luft abgesaugt wird.

Zusätzlich offenbart die US 9,795,731 B2 eine extrakorporale

Blutbehandlungsmaschine mit einer Steuereinheit. Gemäß diesem Dokument wird Luft aus einem venösen Abschnitt eines extrakorporalen Blutkreislaufs mittels einer Blutpumpe entfernt.

Des Weiteren wird auf die aus dem Stand der Technik bekannte WO 2006/122737 A1 verwiesen, die ein Verfahren zum luftfreien Füllen der Blutseite einer Hämodialysevorrichtung mit einer physiologischen Elektrolytlösung offenbart. Zusätzlich ist die US 2013/049974 A1 bekannt, die ein frühes Erkennen eines geringen Bicarbonate-Levels offenbart. Außerdem ist aus dem Stand der Technik die Druckschrift WO 2015/188154 A9 bekannt, die ein System zum Berechnen einer Änderung des Fluids in einer Pumpkammer offenbart.

Grundsätzlich ist es notwendig, extrakorporales Blut zu entgasen, bevor dieses aus dem extrakorporalen Blutkreis einem Patienten zurückgeführt wird. D.h., wenn sich Luft in dem venösen Abschnitt eines extrakorporalen Blutkreislaufs befindet, stellt dieses grundsätzlich eine gefährliche Situation für den Patienten dar. Aus dem Stand der Technik ist es daher bekannt, dass Luft, welche sich in dem venösen Abschnitt des extrakorporalen Blutkreislaufs befindet, entfernt werden muss, wobei eine Blutbehandlungstherapie erst nach der Entfernung fortzusetzen ist.

Daher wird in der Regel der vorstehend genannte Luftabscheider insbesondere in den venösen Leitungsabschnitt des extrakorporalen Blutleitungssystems zwischengeschaltet. Dieser hat normalerweise eine Kammer, in welche Blut bis zu einer bestimmten Füllhöhe eingefüllt wird, derart, dass sich oberhalb des hierbei ausgebildeten Blutspiegels/Pegels ein Hohlraum verbleibt, in welchen die im Blut enthaltene Luft ausgasen und dann über eine Auslassöffnung entweichen kann. Es ist des Weiteren hinlänglich bekannt, dass dieser Ausgasvorgang zum Absinken des Füllstandpegels bzw. des Blutspiegels in der Kammer führt, soweit, dass die Öffnung eines Blut-Einfüllstutzens innerhalb der Kammer oberhalb des abgesunkenen Blutspiegels/Pegels zu liegen kommt. Hierdurch können durch nunmehr eintropfendes Blut wiederum Luftblasen oder Schaum in der Kammer des Luftabscheiders entstehen, wodurch die eigentliche Luftabscheidefunktion beeinträchtigt wird oder ausfällt, was wiederum zu einer Alarmauslösung durch einen der Kammer nachgelagerten Mikroblasendetektor führen kann. Aus dem Stand der Technik ist es daher bekannt, dass zur Lösung dieses Problems auch Pegeldetektoren verwendet werden, welche den aktuellen Pegelstand im Luftabscheider überwachen, derart, dass die Öffnung des Blut-Einfüllstutzens unterhalb des Blutspiegels/Pegels liegt. In US 2016/310657 wird eine Blutbehandlungsvorrichtung beschrieben, die dazu konfiguriert ist, ein Verfahren zur Überwachung des Füllstandes des Blutes in der Vorrichtung durchzuführen, wobei von der Blutpumpe erzeugte Druckpulse mit Referenzwerte der Füllstandshöhe verglichen werden.

Die im Stand der Technik offengelegten Vorrichtungen haben jedoch den Nachteil, dass die Luftentfernung/Luftabscheidung/Entgasung aus dem im venösen Leitungsabschnitt befindlichen Blut relativ kompliziert und umständlich und somit nicht anwenderfreundlich ist. In dem bekannten Stand der Technik werden des Weiteren zahlreiche spezifische Sensoren verwendet, so z.B. aufwändige Luftdetektoren, die erkennen, ob sich für den Patienten gefährliche Luft in dem System befindet, sowie Füllstandsensoren, die erkennen, ob der Füllstand der Luftabscheider-Kammer ausreichend ist. Dies macht die gesamte Sensorik kompliziert und teuer.

Es ist daher die Aufgabe der Erfindung, die Nachteile aus dem Stand der Technik zu vermeiden oder wenigstens zu mildern. Insbesondere soll die Blutbehandlungsmaschine so eingerichtet sein, dass der Pegelstand in der venösen Kammer einfach abgeschätzt werden kann.

Der Kerngedanke der vorliegenden Erfindung besteht im Wesentlichen darin, dass die Blutbehandlungsvorrichtung zusätzlich mindestens einen Druckbestimmungssensor, zur Erfassung des von der Blutpumpvorrichtung eingeleiteten Druckpulses, eine Datenverarbeitungseinheit, die dazu ausgebildet ist, aus dem erfassten Druckpuls einen Füllstandsparameter abzuleiten und in Abhängigkeit von diesem den Zustand eines Benachrichtigungssignals zu ändern und zumindest eine Alarmvorrichtung, die in Abhängigkeit des Zustandes des Benachrichtigungssignals aktiviert wird, aufweist.

Druckpulse, welche durch eine Blutpumpe/Blutpumpenvorrichtung beim Fördern von Blut im extrakorporalen Blutkreis erzeugt und in das geförderte Blut eingegeben werden, zu nutzen/verwenden, um den aktuellen Füllstand zu bestimmen bzw. abzuschätzen.

Hierbei wird der technische Effekt ausgenutzt, dass diese (zwangsläufig erzeugten) Druckpulse innerhalb einer Luftabscheider-Kammer in Abhängigkeit des aktuellen Füllstands und des daraus resultierenden aktuellen Luftpolstervolumens oberhalb des Blutpegels/Blutspiegels innerhalb des Luftabscheiders verändert werden. Diese sensorisch erfassbare(n) Veränderung(en), beispielsweise der Impulsamplitude, lässt/lassen (kontinuierlich) Rückschlüsse auf den aktuellen Füllstand zu.

In anderen Worten ausgedrückt, wird die Aufgabe bei einer gattungsgemäßen Blutbehandlungsvorrichtung dadurch gelöst, dass die Blutbehandlungsvorrichtung mindestens einen Druckbestimmungssensor aufweist, der in der Regel dafür vorgesehen ist, den von einer Blutpumpe/Blutpumpenvorrichtung der Behandlungsvorrichtung erzeugten Blutdruck im venösen Blutleitungssystem insbesondere in der Luftabscheider-Kammer zu erfassen. Erfindungsgemäß wird der Druckbestimmungssensor nunmehr (zusätzlich) zur Erfassung wenigstens eines Füllstandsparameters des von der Blutpumpvorrichtung eingeleiteten Druckpulses eingesetzt/verwendet, wobei eine Datenverarbeitungseinheit vorgesehen ist, die dazu ausgebildet ist, den Zustand eines Benachrichtigungs- oder Warnsignales in Abhängigkeit des von dem Druckbestimmungssensor erfassten wenigstens einen Füllstandsparameters des Druckpulses in einen relevanten Zustand zu ändern, wofür eine Alarmvorrichtung vorgesehen ist, die im Falle eines relevanten Zustandes des Benachrichtigungssignales aktiviert wird.

Das Benachrichtigungssignal kann somit in einer ersten Ausführungsform einen relevanten und einen irrelevanten Zustand annehmen, also zwischen zwei binären Zuständen umschaltbar sein. In einer weiteren Ausführungsform kann das Benachrichtigungssignal kontinuierlich veränderbar sein, also seinen Zustand langsam, also kontinuierlich verändern

Die extrakorporale Blutbehandlungsvorrichtung weist in vorteilhafter Weise den Druckbestimmungssensor auf, der dazu ausgebildet ist, den Füllstand des Kammerbehältnisses/Luftabscheider-Kammer indirekt zu messen. Der Druckbestimmungssensor erfasst somit einen Füllstandsparameter, der einen Wert für den Pegelstand des Kammerbehältnisses darstellt. In weiter vorteilhafter Weise weist die erfindungsgemäße Blutbehandlungsvorrichtung zusätzlich die Datenverarbeitungseinheit auf, die in Abhängigkeit des erfassten Füllstandsparameters den Zustand eines Benachrichtigungssignales in einen relevanten oder einen nicht relevanten Zustand setzt. Durch das Benachrichtigungssignal wird die weiter vorgesehene Alarmvorrichtung in einen aktivierten oder deaktivierten Zustand gesetzt. Es ist somit außer dem (ggf. ohnehin vorhandenen) Druckbestimmungssensor kein weiterer Sensor in der Blutbehandlungsvorrichtung vorgesehen, um den Füllstand im Luftabscheider zu erfassen. In vorteilhafter Weise wird die Blutpumpvorrichtung, die zwangsläufig nötig ist, um das Blut durch die Dialysevorrichtung zu pumpen, zusätzlich genutzt, um den Füllstand innerhalb des Kammerbehältnisses bei einem Absinken wieder zu erhöhen. Dazu muss an der Kammer eine Belüftungsöffnung automatisch geöffnet werden, um die überschüssige Luft gezielt zu entfernen. Stand der Technik sind Rollenpumpen, die Luft entfernen oder hinzufügen können, um den Pegel einzustellen, oder kleine "Kompressoren", die Überdruck zur Verfügung stellen und Ventile zum Luft ablassen.

In einer bevorzugten Ausgestaltung umfasst der wenigstens eine Füllstandsparameter zumindest die Druckimpulsamplitude und/oder die Druckimpulsbreite des von dem Druckbestimmungssensor erfassen Druckpulses. Durch die Bestimmung der Druckimpulsamplitude bzw. der Druckimpulsbreite des von dem Druckbestimmungssensor erfassten Druckpulses kann in vorteilhafter Weise die in dem System befindliche Dämpfung (beeinflusst durch das Luftpolstervolumen im Luftabscheider) ermittelt werden, worüber indirekt der Füllstand des Luftabscheiders bzw. die Luftmenge, die sich in dem Luftabscheider/Kammerbehältnis befindet, ermittelt wird. Die Druckimpulsamplitude und/oder die Druckimpulsbreite sind die charakteristischen Werte, um den Druckpuls zu beschreiben.

In einer weiter bevorzugten Ausführungsform ist die Datenverarbeitungseinheit dazu ausgebildet, zur Ermittlung der Druckimpulsamplitude (nachfolgend nur noch als Druckamplitude bezeichnet) und der Druckimpulsbreite (nachfolgend nur noch als Druckbreite bezeichnet) aus dem Druckpuls ein Frequenzanalyseverfahren, vorzugsweise mittels Fouriertransformation, auszuführen. Bei diesem Frequenzanalyseverfahren werden die periodischen Funktionen in eine Summe von Winkelfunktionen aufgespalten. Die Periodendauer einer einzelnen Schwingung bzw. einer Oberwelle beträgt dann ein ganzzahliges Vielfaches einer Grundschwingung mit der Periodendauer und der Kreisfrequenz. Die Amplituden dieser Schwingungen bezeichnet man als Fourierkoeffizienten. Unter mathematischen Gesichtspunkten handelt es sich dabei um eine Reihenentwicklung. Mit diesem Verfahren ergibt sich in vorteilhafter Weise die Möglichkeit, ein gemessenes Signal durch seine einzelnen Frequenzanteile darzustellen und man erhält ein Frequenzspektrum. Durch die Umwandlung der Sinus- bzw. Cosinus-Anteile gleicher Frequenz in Polarkoordinaten entsteht ein Amplituden- und ein Phasenspektrum.

Weiter vorteilhaft stellt der Füllstandsparameter ein Maß für den Füllstand des Blutes in dem Kammerbehältnis dar und die Datenverarbeitungseinheit ist dazu ausgebildet, das Benachrichtigungssignal in einen relevanten Zustand zu ändern, sobald der Füllstandsparameter einen vordefinierten Füllstandsgrenzwert unterschreitet. Durch die Ermittlung des Füllstandsparameters wird die Druckamplitude und die Druckbreite des von dem Druckbestimmungssensor erfassten Druckpulses ermittelt, wodurch das Luftvolumen in dem Kammerbehältnis mittels der Dämpfung abgeschätzt wird. Falls der Füllstandsparameter einen vordefinierten Füllstandsgrenzwert unterschreitet, also der Pegel in dem Kammerbehältnis unter eine definierte Marke sinkt, wird das Benachrichtigungssignal in einen relevanten - also einen kritischen Zustand - versetzt.

In einer weiteren Ausgestaltungsform ist die Datenverarbeitungseinheit zusätzlich dazu ausgebildet ein Alarmierungssignal in einen relevanten Zustand zu ändern und zumindest die Alarmvorrichtung zu aktivieren, sobald ein vordefinierter Häufigkeitsgrenzwert, der definiert wie häufig der Füllstand des Blutes in dem Kammerbehältnis in einem vordefinierten Zeitintervall den Füllstandsgrenzwert unterschreitet, überschritten wird. In vorteilhafter Weise wird die Blutpumpvorrichtung so angesteuert, dass diese den Blutpegel innerhalb des Kammerverhältnisses erhöht, sobald der Pegel des Blutes in dem Kammerbehältnis unterschritten wird. Somit ist in vorteilhafter Weise bewirkt, dass keine Luftblasen in das Blutleitungssystem eingeführt werden.

Weiter vorteilhaft weist der Druckbestimmungssensor eine Abtastrate von zumindest 100Hz auf, um den zeitlichen Verlauf des Druckpulses des Blutes zu erfassen. Dabei handelt es sich vorzugsweise um einen normalen venösen Drucksensor, der entweder über TP (Transducer Protector Filter) oder über PODs indirekt den Druck in dem Blutleitungs-system aufnimmt.

In einer weiter vorteilhaften Ausführungsform weist die Blutpumpvorrichtung zusätzlich eine Pegel- und/oder Level-Regulierungspumpe auf. Durch diese Ausgestaltung ist eine weitere Pumpe vorgesehen, die dazu ausgebildet ist, das Level bzw. den Pegel in dem Blutleitungssystem bzw. in dem Kammerbehältnis konstant zu halten, so dass der Pegel nicht unter einen kritischen Wert sinkt.

Weiter vorteilhaft ist ein Verfahren zur Überwachung des Füllstandes des Blutes in einer erfindungsgemäßen Blutbehandlungsvorrichtung beansprucht, welches die folgenden Schritte umfasst:

- Erfassen des von der Blutpumpvorrichtung eingeleiteten Druckpulses durch den Druckbestim m ungssensor;
- Ableiten eines Füllstandsparameters aus dem von dem Druckbestimmungssensor erfassten Druckpulses,
- kontinuierliches Analysieren und Verarbeiten des erfassten Füllstandsparameters durch die Datenverarbeitungseinheit;
- Verändern des Zustandes eines Benachrichtigungssignals in Abhängigkeit des Füllstandsparameters, sobald dieser einen vordefinierten Füllstandsgrenzwert unterschreitet und
- Aktivieren der Alarmvorrichtung in Abhängigkeit des Zustandes des Benachrichtigungssignals.

Die grundsätzliche Idee der erfindungsgemäßen Vorrichtung besteht darin, das Luftvolumen in dem Kammerbehältnis mittels der ermittelten Dämpfung abzuschätzen, wobei sich die Dämpfung verändert, wenn sich Schaum oder Luft in dem Blutleitungssystem oder dem Kammerbehältnis befindet. Es wird somit in vorteilhafter Weise kein zusätzlicher Füllstandsensor benötigt, sondern die erfassten Füllstandsparameter werden indirekt über die Auswertung des Druckpulses der Blutpumpvorrichtung ermittelt, da der Druckpuls sowieso in dem Blutleitungssystem der Blutbehandlungsvorrichtung vorhanden ist. Die Menge von Schaum wird somit auch erkannt und bestimmt.

In einem weiter vorteilhaften Verfahrensschritt wird ein kontinuierliches Ermitteln der Druckamplitude und/oder der Druckbreite aus dem Druckpuls mittels eines Frequenzanalyseverfahren durch die Datenverarbeitungseinheit beansprucht. Die Datenverarbeitungseinheit ist somit ein kontinuierlich arbeitender Prozessor in Kombination mit einer Speichereinheit, welche die bereitgestellten Daten, Parameter, Referenzwerte und die vordefinierten Grenzwerte kontinuierlich erfasst, diese auswertet, bewertet und an andere Einheiten, beispielsweise die Alarmvorrichtung, bereitstellt.

Weiter vorteilhaft umfasst das Verfahren die folgenden Schritte:

- Verändern des Benachrichtigungssignals in einen relevanten Zustand, sobald der Füllstandsparameter einen vordefinierten Füllstandsgrenzwert unterschreitet;
- Erzeugen eines Ansteuersignales im Falle eines relevanten Zustandes des Benachrichtigungssignals und
- Ansteuern der Blutpumpvorrichtung, so dass der Füllstand des Blutes in dem Kammerbehältnis erhöht wird, sobald der vordefinierte Füllstandsgrenzwert unterschritten wird.

Sobald der Füllstandsgrenzwert, also der Pegelstand des Blutes in dem Kammerbehältnis, einen vordefinierten Füllstandsgrenzwert, also einen vordefinierten Grenzwert, der bei Unterschreitung des Blutpegels eine Gefahr für den Patienten darstellen würde, unterschreitet, wird ein Benachrichtigungssignal von der Datenverarbeitungseinheit generiert. Aus diesem Benachrichtigungssignal generiert die Datenverarbeitungseinheit wiederum ein Ansteuersignal, welches die Blutpumpvorrichtung und die Pegelanpassugnsvorrichtung, so ansteuert, dass diese den Füllstand des Blutes in dem Kammerbehältnis erhöht, so dass die Gefahr von dem Patienten abgewendet wird, dass Luft in sein Blut gelangt und er dadurch geschädigt wird. Insgesamt ist durch das erfindungsgemäße Verfahren somit ein Regelungsverfahren bereitgestellt, welches kontinuierlich dafür sorgt, dass ausreichend Blut in dem Blutleitungssystem und dem Kammerbehältnis vorhanden ist, welches ausreichend Luft-frei ist und welches solch einen hohen Pegelstand aufweist, dass der Patient kontinuierlich mit ausreichend Blut versorgt wird.

Weiter vorteilhaft umfasst das Verfahren die folgenden Schritte:

- Ansteuern der Pegel- und/oder Level-Regulierungspumpe und der Belüftungsöffnung des Kammerbehältnisses, sobald der vordefinierte Füllstandsgrenzwert unterschritten wird. Die Pegel- bzw. die Level-Regulierungspumpe ist somit zusätzlich dazu ausgebildet, den Füllstand in dem Kammerbehältnis auf einem vordefinierten Pegel bzw. Level zu halten. Des Weiteren wird die Belüftungsöffnung geöffnet, um überschüssige Luft zu entfernen, so dass der gesamte Blutkreislauf entlüftet wird.

In einer weiter vorteilhaften Ausgestaltungsform umfasst das Verfahren die folgenden Schritte:

- Verändern des Zustandes eines Alarmsignals in einen relevanten Zustand, sobald der Füllstandsgrenzwert einen vordefinierten Häufigkeitsgrenzwert überschreitet und
- Aktivierung der Alarmvorrichtung im Falle eines relevanten Zustandes des Alarmsignals.

Wenn das System somit gezwungen ist, den Füllstand des Blutes häufig nachzuregeln, also häufig Luft in dem Blut vorhanden ist, wird ein Alarmsignal ausgelöst, welches die Alarmvorrichtung aktiviert. In diesem Fall ist das System von einer Undichtigkeit, einer Leckage oder einem weiteren technischen Problem betroffen, über welches das Fachpersonal zu informieren ist. Wenn Luft in dem Blutleitungssystem erfasst wird, stoppt die Datenverarbeitungseinheit die Blutpumpe und gibt über die Alarmvorrichtung ein Alarmsignal aus.

Mit anderen Worten betrifft die Erfindung eine Dialysemaschine, die einen Drucksensor aufweist, wobei dieser den Druckverlauf, der den Druck in der Kammer bestimmt, hochaufgelöst misst. Die Frequenz ist so gewählt, dass Druckpulse, die durch die Peristaltik der Blutpumpe entstehen, noch gut aufgelöst werden können. Der Druckpuls, die Höhe, die Breite und die Dämpfung werden mittels Frequenzanalyse, insbesondere Fouriertransformation, bestimmt. Ein kleines Volumen bedeutet, dass das Kammerbehältnis voll ist. Dies bedeutet wiederum einen höheren Frequenzanteil, also einen höheren und kürzeren Puls. Ein großes Volumen bedeutet, dass das Kammerbehältnis leer ist, also ein höherer Grundanteil der Frequenz und insgesamt ein gedämpfter und verzögerter Verlauf des Signals. Eine Referenzwert ist dabei entweder fest gespeichert oder wird erst nach dem Befüllen (voll) definiert.

Bei einer Abweichung größer X, also bei einer Veränderung des Spektrums und der Amplituden über Grenzen hinaus, wird alarmiert, bevor die Kammer ganz leer ist. Alternativ ist es auch möglich, dass der Pegel automatisch nachgeregelt wird. Falls dieses Nachregeln zu oft erfolgt, erfolgt trotzdem eine Alarmierung, da es irgendwo einen Lufteintritt gibt bzw. das System undicht bzw. geschädigt ist. In einer weiteren Alternative wird über den Anschluss zum Pegelheben- oder zum Pegelsenken bei bestehender Blutpumpe und geschlossener venöser Klemme eine bestimmte, bekannte Menge Luft eingebracht. Aus dem daraus resultierenden Druckanstieg kann direkt das Volumen der Luft in der Kammer bestimmt werden. Dies kann mittels der Vorrichtung zum Pegelheben- und Pegelsenken erfolgen, die über eine Pumpe verfügt. Zusätzlich ist vorgesehen, bestimmte Bloodlines zu erkennen, da sich das Spektrum abhängig von der Kammergröße und dem Kammermaterial ändert.

Bei dem Drucksensor handelt es sich um einen normalen venösen Drucksensor, der entweder über TP (Transduder Protector Filter) oder über PODs indirekt den Druck in der venösen Leitung aufnimmt. Die Abtastrate des Sensors muss in den Bereich von ca. 100Hz Abtastrate liegen, um die Pulse ausreichend genau aufzulösen.

Die Verfahrensschritte der Schaumerkennung funktionieren so, dass das Luftvolumen in der Kammer mittels der Dämpfung abgeschätzt wird, da sich Schaum anders verhält als reines Blut bzw. einen anderen Dämpfungskoeffizienten aufweist.

Vorzugsweise ist die Datenverarbeitungseinheit dafür angepasst ist, aus dem erfassten Druckpuls eine Dämpfung des Druckpulses als weiteren Füllstandsparameter zu bestimmen und mit einem in der Datenverarbeitungseinheit hinterlegten zugehörigen Referenzwert zu vergleichen.

Gemäß einer weiteren bevorzugten Ausführungsform kann als Referenzwert eine maximale Druckimpulsbreite und/oder eine minimale Druckimpulsamplitude hinterlegt sein; oder es kann eine minimale Druckimpulsbreite und/oder eine maximale Druckimpulsamplitude hinterlegt sein und bei Vergleich und Unterschreiten des minimalen Werts und/oder Überschreiten des maximalen Werts die Datenverarbeitungseinheit den Zustand des Benachrichtigungssignals entsprechend ändern. Auf diese Weise kann etwa eine maximale Druckimpulsbreite als Grenzwert dienen, so dass bei Überschreiten dieses Grenzwerts die Datenverarbeitungseinrichtung erkennt und bestimmt dass eine Füllstandshöhe zu niedrig ist. Da etwa eine hohe Füllstandshöhe des Blutes in der Kammer eine geringere Druckimpulsbreite als eine niedrige Füllstandshöhe aufweist, kann so auf Basis des zugehörigen Füllstandsparameters bei Über- bzw. Unterschreiten eines vorgegebenen Grenzwerts auf die zugehörige Füllhöhe, insbesondere auf die zugehörige untere Füllstandsgrenze für einen sicheren Betrieb der Tropfkammer geschlossen werden.

Vorzugsweise kann die Datenverarbeitungseinheit zusätzlich dafür angepasst sein, den Zustand des Benachrichtigungssignals zu ändern und zumindest die Alarmvorrichtung zu aktivieren, sobald ein vordefinierter Häufigkeitsgrenzwert, der definiert wie häufig der Füllstand des Blutes in dem Kammerbehältnis in einem vordefinierten Zeitintervall einen Füllstandsgrenzwert unterschreitet, überschritten wird.

Für eine (Druck-)Impulsbreite gibt es verschiedene Definitionen. Eine Definition der Pulsbreite bezieht sich beispielsweise auf die Zeit eines Pulses, die zwischen den 50-%-Werten von der ansteigenden und fallenden Pulsflanke liegt. Der 50-%-Wert, Full Width at Half Maximum (FWHM), bezieht sich auf den maximalen Amplitudenwert des Pulses. Eine andere Definition etwa benutzt den 90-%-Wert oder 1/e, mit e=2,718 des maximalen Amplitudenwertes.

Eine Frequenz-Amplitude ist eine Amplitude einer Frequenz in dem Frequenzspektrum. Eine Frequenz-Bereichs-Amplitude ist eine Amplitude eines vorbestimmten Bereichs der Frequenzen, etwa von einer festgelegten minimalen Frequenz bis zu einer festgelegten maximalen Frequenz.

Mit anderen Worten kann ein Verfahren zur Überwachung des Füllstandes des Blutes in einer Blutbehandlungsvorrichtung insbesondere die folgenden Schritte umfassen: Erfassen des von der Blutpumpvorrichtung eingeleiteten Druckpulses durch den Druckbestimmungssensor; Ableiten eines Füllstandsparameters aus dem von dem Druckbestimmungssensor erfassten Druckpuls, kontinuierliches Analysieren und Verarbeiten des erfassten Füllstandsparameters durch die Datenverarbeitungseinheit; Verändern des Zustandes eines Benachrichtigungssignals in Abhängigkeit des Füllstandsparameters, sobald dieser einen vordefinierten Füllstandsgrenzwert unterschreitet und Aktivieren der Alarmvorrichtung in Abhängigkeit des Zustandes des Benachrichtigungssignals.

Gemäß einer Ausführungsform kann das Verfahren die folgenden Schritte aufweisen: Verändern des Benachrichtigungssignals in einen relevanten Zustand, sobald der Füllstandsparameter einen vordefinierten Füllstandsgrenzwert unterschreitet; Erzeugen eines Ansteuersignales im Falle eines relevanten Zustandes des Benachrichtigungssignals und Ansteuern der Blutpumpvorrichtung, so dass der Füllstand des Blutes in dem Kammerbehältnis erhöht wird, sobald der vordefinierte Füllstandsgrenzwert unterschritten wird.

Vorzugsweise kann das Verfahren den Schritt aufweisen: Ansteuern der Pegel- und/oder Level-Regulierungspumpe und der Belüftungsöffnung des Kammerbehältnisses, sobald der vordefinierte Füllstandsgrenzwert unterschritten wird.

Die Erfindung wird nachfolgend mit Hilfe der folgenden Figuren weiter erläutert. Es zeigen:

- Fig. 1 eine Blutbehandlungsvorrichtung gemäß der vorliegenden Erfindung mit einem näherungsweise vollen Kammerbehältnis;
- Fig. 2 eine Blutbehandlungsvorrichtung gemäß der vorliegenden Erfindung mit einem näherungsweise leeren Kammerbehältnis;
- Fig. 3 zeigt in den Unterfiguren Fig. 3a, Fig. 3b und Fig. 3c eine Übersicht über Eingangs- und Ausgangsparameter des Frequenzanalyseverfahrens;

- Fig. 4 zeigt in den Unterfiguren Fig. 4a und Fig. 4b eine Übersicht über Eingangs- und Ausgangsparameter des Frequenzanalyseverfahrens;
- Fig. 5 zeigt verschiedenen Frequenzspektren, die entsprechende Füllstände der jeweiligen Kammerbehältnisse repräsentieren und
- Fig. 6 zeigt die von der Datenverarbeitungseinheit verarbeiteten Grenzwerte und Parameter und die Signale, die an die jeweiligen Ausgabevorrichtungen gesendet werden.

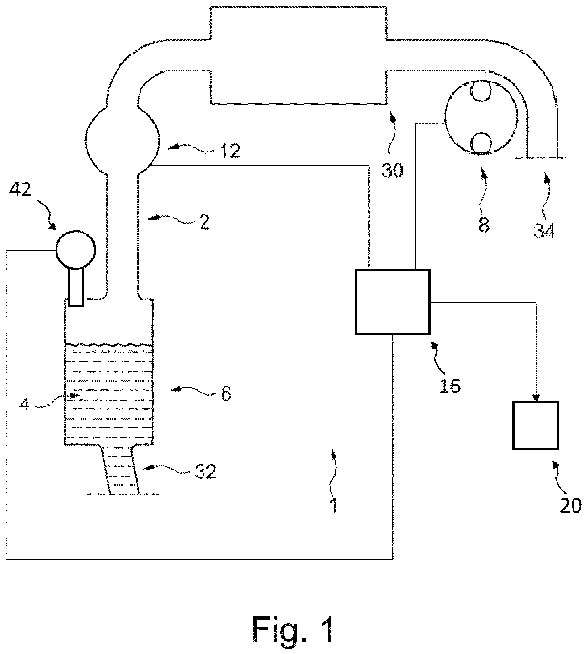

Die Fig. 1 zeigt eine Blutbehandlungsvorrichtung 1, die ein Blutleitungssystem 2 umfasst, welches Blut 4 in seinem Kammerbehältnis 6 aufweist. Es ist deutlich zu erkennen, dass das Kammerbehältnis 6 zu ca. dreiviertel des maximalen Füllstandes mit Blut 4 gefüllt ist. Des Weiteren weist die Blutbehandlungsvorrichtung 1 eine Blutpumpvorrichtung 8 auf, vorzugsweise eine Schlauchpumpe, welche das Blut 4 in dem Blutleitungssystem 2 umpumpt. Des Weiteren ist ein Druckbestimmungssensor 12 zu erkennen, der in dem Blutleitungssystem 2 integriert ist. Der Druckbestimmungssensor 12 kann aber auch an dem Kammerbehältnis 6 vorgesehen sein. Weiterhin ist der Eingang 32 des Kammerbehältnisses 6 und der Ausgang 34 des Blutleitungssystems 2 zu erkennen. Die gezeigte Blutbehandlungsvorrichtung 1 zeigt den Druckbestimmungssensor 12 der signalleitend mit der Datenverarbeitungseinheit 16, wobei die Signalleitung sowohl unidirektional als auch bidirektional stattfinden kann, also der Druckbestimmungssensor 12 erfasste Signale an die Datenverarbeitungseinheit 16 sendet, diese aber auch Signale an den Druckbestimmungssensor 12 zurücksenden kann. Des Weiteren ist gezeigt, dass sowohl die Blutpumpvorrichtung 8 als auch die Alarmvorrichtung 20 signalleitend mit der Datenverarbeitungseinheit 16 verbunden sind. Die Signalleitung kann in sämtlichen Fällen sowohl uni- als auch bidirektional erfolgen. Die Alarmvorrichtung 20 kann beispielsweise auch als Mobiles-Kommunikationsgerät, bspw. Tablet und/oder Handy, ausgebildet sein.

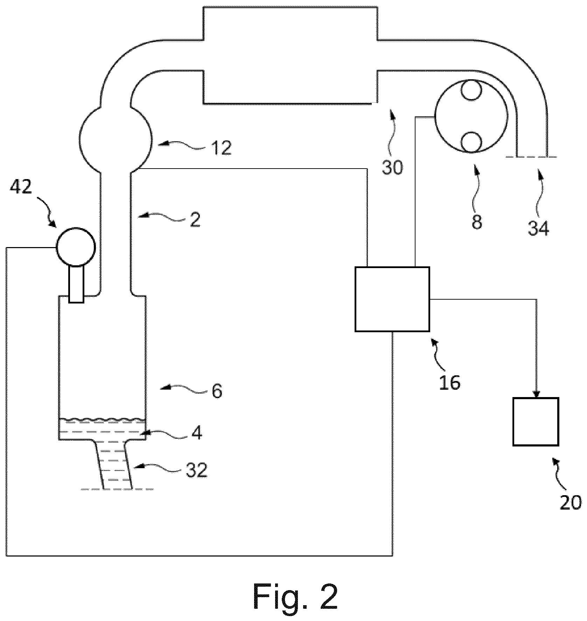

Die Fig. 2 zeigt einen weiteren Zustand der Blutbehandlungsvorrichtung 1, in welchem das Kammerbehältnis 6 näherungsweise leer und nicht mit Blut 4 gefüllt ist. Es ist wiederum die Blutpumpvorrichtung 8 und der Druckbestimmungssensor 12 zu erkennen, der in dem Blutleitungssystem 3 integriert ist. Das Blutleitungssystem 2 weist wiederum einen Eingang 32 und einen Ausgang 34 auf. Fig. 2 zeigt auch die signalleitende Anbindung der Datenverarbeitungseinheit 16 an die Blutpumpvorrichtung 8, den Druckbestimmungssensor 12 und die Alarmvorrichtung 20. Die Blutpumpvorrichtung 8 kann zusätzlich eine Pegel- und/oder eine Level-Regulierungspumpe 42 aufweisen, die explizit dafür ausgebildet ist, den Füllstand auf einem vorgegebenen Niveau zu halten.

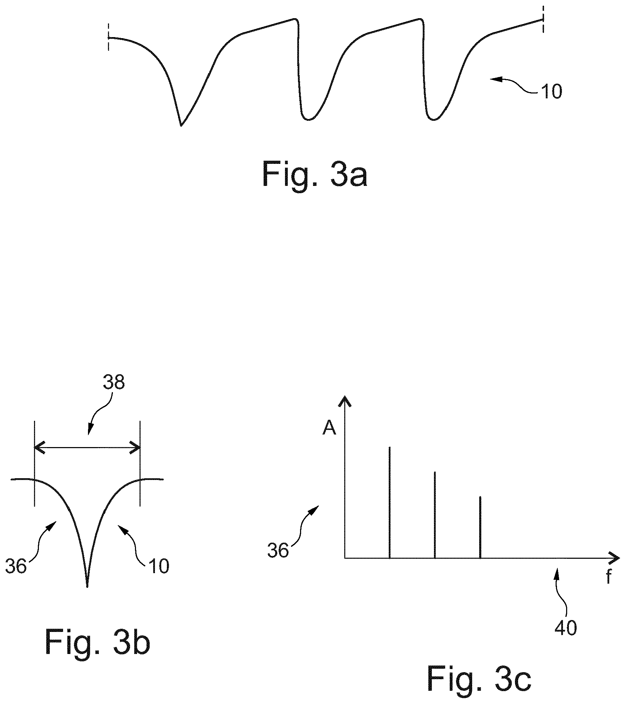

Die Fig. 3 ist in die einzelnen Fig. 3a, 3b und 3c gegliedert. Die Fig 3a zeigt einen zeitlichen Druckverlauf des Druckpulses 10, der so von der Blutpumpvorrichtung 8 oder einer weiteren Druckpulsquelle bereitgestellt wird. Die weitere Druckpulsquelle kann beispielsweise auch extern angeordnet sein. Die Fig. 3b zeigt einen Druckpuls 10, der so von dem Druckbestimmungssensor 12 gemessen wurde und von der Datenverarbeitung 16 verarbeitet wird. Der gemessene Druckpuls 10 weist eine Druckamplitude 36 und eine Druckbreite 38 auf. Die Fig. 3c zeigt ein Frequenzspektrum, welches auf der Ordinate eine Druckamplitude 36 aufgetragen hat und auf ihrer Abszisse die Frequenz 40 aufgetragen hat. Die Fig. 3b und die Fig. 3c stehen dabei beispielhaft für ein voll befülltes Kammerbehältnis, da der Druckpuls 10 "scharf" ausgeprägt ist und eine große Druckamplitude 36 aufweist. Dieses ist auch in dem der hohen Druckamplitude 36 des Frequenzspektrums zu erkennen.

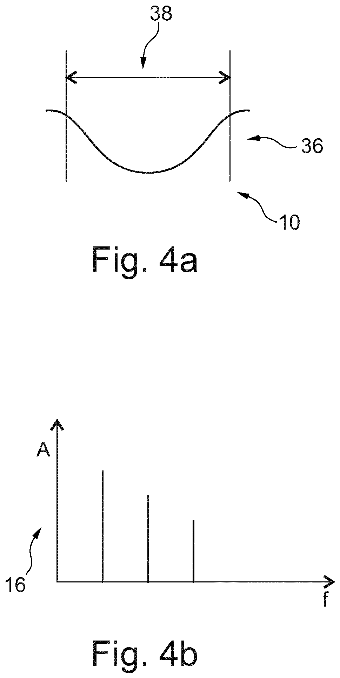

Die Fig. 4 ist in die Fig. 4a und die Fig. 4b gegliedert. Die Fig. 4a zeigt einen von dem Druckbestimmungssensor 12 gemessenen Druckpuls 10, der einen breiten Druckpuls 38 mit einer geringen Druckamplitude 36 aufweist. Der gemessene Druckpuls wird durch das Frequenzspektrum, welches in der Fig. 4b gezeigt ist, repräsentiert, wobei auf der Ordinate wiederum die Druckamplitude 36 aufgezeigt ist und auf der Abszisse die Frequenz f gezeigt ist.

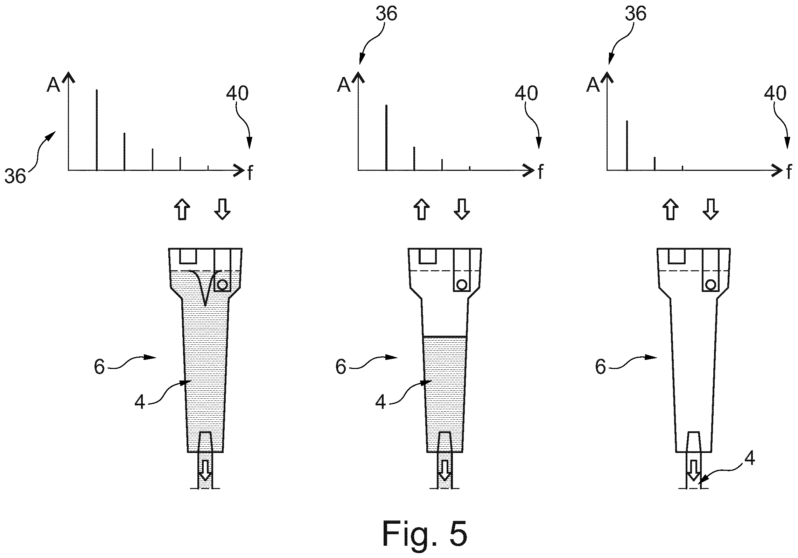

Die Fig. 5 zeigt drei unterschiedliche Füllzustände des Kammerbehältnisses 6. In dem ersten Füllzustand ist das Kammerbehältnis 6 vollständig mit Blut 4 gefüllt. Diesem ist ein darüber liegendes Frequenzspektrum zugeordnet, welches auf seiner Ordinate wiederum die Druckamplitude 36 aufweist und auf der Abszisse wiederum die Frequenz 40 aufweist. Es ist zu erkennen, dass die Druckamplitude 36 des ersten Frequenzspektrums sehr stark ausgeprägt ist und mit steigender Frequenz 40, also mit Frequenzspektren, die sich weiter links befinden, langsam abfällt. In dem mittleren Füllzustand ist das Blut 4 in dem Kammerbehältnis 6 zur Hälfte gefüllt und das rechte Kammerbehältnis 6 ist blutleer. Es ist wiederum zu erkennen, dass in dem mittleren Füllzustand die Druckamplitude 36 bereits geringer ausgeprägt ist als in dem vollständig gefüllten Zustand, der links dargestellt ist. In dem vollständig geleerten Zustand, ganz rechts dargestellt, ist die Druckamplitude 36 noch geringer ausgeprägt. Es ist somit offensichtlich, dass das Frequenzspektrum eine eindeutige Zuordnung zu dem jeweiligen Füllzustand des Kammerbehältnisses 6 gestattet.

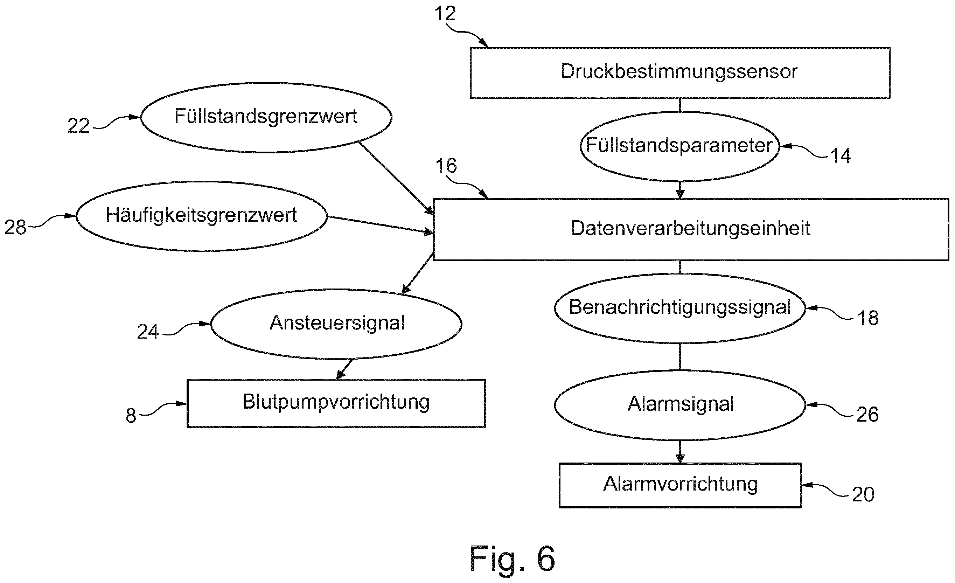

Die Fig. 6 zeigt eine Datenverarbeitungseinheit 16, die einen Füllstandsparameter 14 von dem Druckbestimmungssensor 12 zugespielt bekommt. Die Datenverarbeitungseinheit 16 verarbeitet den erhaltenen Füllstandsparameter 14 unter Berücksichtigung des Füllstandsgrenzwertes 22 zu einem Benachrichtigungssignal 18, welches in einen aktivierten oder einen deaktivierten Zustand gesetzt wird, bzw. in ein Alarmsignal 26, welches auch in einen aktivierten oder deaktivierten Zustand versetzbar ist, um die Alarmvorrichtung 20 zu aktivieren. Des Weiteren wird der Datenverarbeitungseinheit 16 ein Häufigkeitsgrenzwert 28 vorgegeben, der definiert, wie oft die Blutpumpvorrichtung 8 das Kammerbehältnis 6 wieder befüllen darf, um sich noch in einem beschädigungsfreien Zustand zu befinden und keine Leckage aufweist. Falls der Häufigkeitsgrenzwert 28 überschritten wird, wird ein Alarmsignal 26 ausgegeben, um die Alarmvorrichtung 20 zu aktivieren. Falls der Füllstand des Kammerbehältnisses/Luftabscheiders den Füllstandsgrenzwert 22 unterschreitet, wird ein Steuersignal 24 generiert, welches dazu ausgebildet ist, die Pumpvorrichtung 8 zu aktivieren, das Kammerbehältnis 6 wieder mit Blut 4 zu befüllen.

Im Folgenden werden die Verfahrensschritte genannt, die nötig sind, um zu erkennen, wie sich das Frequenzspektrum in Abhängigkeit der Kammergröße und des Kammermaterials im Detail ändert (dieses ist auch unter dem Begriff bekannt, dass verschiedene "Bloodlines" erkannt werden).

Die grundsätzliche Idee an dem erfindungsgemäßen Verfahren ist, vor dem Priming, d.h. wenn nur Luft in der Leitung ist, die venöse Klemme zu schließen und dann mittels der Blutpumpe eine feste Menge Luft hineinzupumpen. Dazu kann die Blutpumpvorrichtung (Pumpe) vorher in eine Stellung gebracht werden, die es ermöglicht, bspw. genau eine Umdrehung mit bekannter Fördermenge zu fördern. In diesem Fall ist die Startposition der Rolle gerade noch im Eingriff auf der Pumpenausgangsseite. Die zu pumpender Blutmenge beträgt ca. 10-15 ml je Umdrehung abhängig von der jeweiligen Blutpumpvorrichtung. Die venöse Kammer (20-40 ml), der Dialysator (80-120 ml) und der Schlauch (ca. 10 ml) haben zusammen ein Volumen von ca. 110-170 ml. Bei einem bekannten Dialysator lässt sich aus dem Druckanstieg das Volumen der Kammer errechnen und bei einem bekannten Blutleitungssystem lässt sich das blutseitige Fühlvolumen des Dialysators berechnen. Grundlage hierfür ist, dass die Zustandsgleichung idealer Gase gilt, die besagt, dass das (Druck x Volumen) /Temperatur=Konstant ist, oder mathematisch ausgedrückt: Px x V /Temp = konst. Es ist das Gesamtvolumen des Dialysators+ Kammer zu ermitteln, daraus lässt sich dann das Priming Volumen ermitteln und optimieren. Das ermittelte Volumen zusätzlich einer Annahme für den restlichen Schlauch beträgt typischerweise 4 x 25ml/m = 100ml/m + ermitteltes Volumen für die Kammer und für den Dialysator. Es gelten die im Folgenden aufgeführten Formeln, mit folgender Nomenklatur und den folgenden Annahmen:

- Vk = Kammervolumen
- Vd = Dialysatorvolumen
- VL = Luftbolus zur Druckerhöhung
- P = Druck in dem jeweiligen Volumen
- Annahme: Das Volumen innerhalb des Schlauchs wird vernachlässigt

Es ist folgender Formelapparat anzuwenden:

$$P1x(V1+VL)/T= P2x(V1)/T$$

$$V1 = Vd+Vk+VL$$

- P1V1 +P1VL = P2V1 (wobei die Annahme gilt: temp = konst; V1+VL wird auf V1 komprimiert)

$$P1VL = P2V1-P1V1$$

$$P1VL/V1 = P2-P1$$

$$V1 = P1VL(P2-P1)$$

$$Vd+Vk+VL= P1VL/(P2-P1)$$

$$Vk = P1VL/(P2-P1)-Vd-VL$$

Des Weiteren kann die Behandlungsvorrichtung 1 die im folgenden aufgeführten Überwachungsfunktionen, Vorrichtungsmerkmale oder Zustände aufweisen:

Eine Füllstandsüberwachung des Luftabscheiders einer Dialysemaschine, eine Vorrichtung zur Bestimmung des Füllstandes des Luftabscheiders über den Druckverlauf, eine Vorrichtung zur Bestimmung des arteriellen bzw. venösen Druckverlaufes, ein Verfahren zur Bestimmung des Füllstandes des Luftabscheiders, ein Verfahren zur Bestimmung des arteriellen bzw. venösen Druckverlaufes, die Ermittlung des Füllstandes durch Frequenzanalyse des Druckverlaufes, die Bestimmung des Füllstandes durch Einbringung einer bestimmten Menge Luft oder Flüssigkeit und Auswertung des Druckanstieges, die Einbringung der Luft durch Anschlüsse Pegelheben-/Pegelsenken bei stehender Blutpumpe und geschlossenem SAKV, die Einbringung der Luft oder Flüssigkeit durch die Blutpumpe bei geschlossenem SAKV, der Vergleich des Druckverlaufes mit einem Referenzzustand, den Referenzzustand als fixierten Wert speichern, die Bestimmung des Referenzzustandes im gefüllten Zustand, die Alarmierung bei bestimmter Abweichung vom Referenzzustand, die automatische Nachregelung des Füllzustandes bei der Abweichung vom Referenzzustand, die Alarmierung bei zu häufiger Nachregelung des gefüllten Zustandes, eine Vorrichtung zur Erkennung von Schaum im Luftabscheider, eine Vorrichtung zur Bestimmung der Menge von Schaum und Blut, ein Verfahren zur Erkennung von Schaum im Luftfahrtschneider, ein Verfahren zur Bestimmung der Menge von Schaum im Blut und/oder eine Vorrichtung zur Erkennung der verwendeten Blutschlauchsysteme.

## Bezugszeichenliste

1) Blutbehandlungsvorrichtung
2) Blutleitungssystem
4) Blut
6) Kammerbehältnis
8) Blutpumpvorrichtung
10) Druckpuls
12) Druckbestimmungssensor
14) Füllstandsparameter
16) Datenverarbeitungseinheit
18) Benachrichtigungssignale
20) Alarmvorrichtung
22) Füllstandsgrenzwert
24) Ansteuersignal
26) Alarmierungssignal
28) Häufigkeitsgrenzwert
30) Dialysevorrichtung
32) Eingang
34) Ausgang
36) Druckamplitude
38) Druckbreite
40) Frequenz
42) Pegel- bzw.- Level-Regulierungspumpe

## Patentansprüche

1. Blutbehandlungsvorrichtung (1) zur extrakorporalen Blutbehandlung, aufweisend ein Blutleitungssystem (2) mit einem Kammerbehältnis (6) zur Abscheidung von Blasen aus einem zu behandelnden Blut (4), und einer Blutpumpvorrichtung (8), die dazu ausgebildet ist, das Blut (4) zu pumpen und die zusätzlich Druckpulse (10) mit einer vordefinierten Frequenz in dem Blutleitungssystem (2) erzeugt,
**dadurch gekennzeichnet, dass**
die Blutbehandlungsvorrichtung (1) zusätzlich aufweist:

- einen Druckbestimmungssensor (12) in dem Blutleitungssystem (2) zur Erfassung des von der Blutpumpvorrichtung (8) eingeleiteten Druckpulses (10),
- eine Datenverarbeitungseinheit (16), die dazu ausgebildet ist, aus dem erfassten Druckpuls (10) eine Druckimpulsamplitude (36) und/oder eine Druckimpulsbreite (38) des Druckpulses (10) zu bestimmen, die als Füllstandsparameter (14) dienen, die Füllstandsparameter (14) Druckimpulsbreite (38) und/oder Druckimpulsamplitude (36) mit zumindest einem in der Datenverarbeitungseinheit (16) hinterlegten Referenzwert zu vergleichen, anhand des Vergleiches mit dem Referenzwert eine Füllstandshöhe des Blutes (4) in dem Kammerbehältnis (6) zu bestimmen und in Abhängigkeit von den Füllstandsparametern (14) und damit der Füllstandshöhe des Blutes (4) in dem Kammerbehältnis (6) einen Zustand eines Benachrichtigungssignals (18) zu ändern,

- zumindest eine Alarmvorrichtung (20), die in Abhängigkeit des Zustandes des Benachrichtigungssignals (18) aktiviert wird.

2.  Blutbehandlungsvorrichtung (1) nach Anspruch 1, **dadurch gekennzeichnet, dass** als Referenzwert der erfasste Druckpuls (10) nach einem ersten Befüllen des Kammerbehältnisses (6) mit dem Blut (4) oder ein zuvor bestimmter Druckpuls, der einen vollständig mit Blut (4) gefüllten Zustand des Kammerbehältnisses (6) darstellt, in der Datenverarbeitungseinheit (16) hinterlegt ist, und bei einer vorbestimmten maximalen Abweichung zwischen der Druckimpulsbreite (38) des erfassten Druckpulses (10) und der Druckimpulsbreite des hinterlegten Referenzwerts und/oder einer vorbestimmten maximalen Abweichung zwischen der Druckimpulsamplitude (36) des erfassten Druckpulses (10) und der Druckimpulsamplitude des Referenzwerts die Datenverarbeitungseinheit (16) den Zustand des Benachrichtigungssignals (18) ändert.

3.  Blutbehandlungsvorrichtung (1) nach Anspruch 1, **dadurch gekennzeichnet, dass** als Referenzwert ein erfasster Druckpuls (10) des Kammerbehältnisses (6) bei einem vordefinierten Füllstandsgrenzwert (22) in der Datenverarbeitungseinheit (16) hinterlegt ist und bei einer vorbestimmten minimalen Abweichung zwischen der Druckimpulsbreite (38) des erfassten Druckpulses (10) und der Druckimpulsbreite des hinterlegten Referenzwerts oder eines Überschreitens und/oder einer vorbestimmten minimalen Abweichung zwischen der Druckimpulsamplitude (36) des erfassten Druckpulses (10) und der Druckimpulsamplitude des Referenzwerts oder eines Unterschreitens die Datenverarbeitungseinheit (16) den Zustand des Benachrichtigungssignals (18) ändert.

4.  Blutbehandlungsvorrichtung (1) nach einem der vorstehenden Ansprüche, **dadurch gekennzeichnet, dass** die Blutbehandlungsvorrichtung (1) in seinem Blutleitungssystem (2) genau einen Druckbestimmungssensor (12) aufweist und mit diesem einen Druckbestimmungssensor (12) in der Lage ist, die Füllstandshöhe des Blutes (4) in dem Kammerbehältnis (6) zu bestimmen.

5.  Blutbehandlungsvorrichtung (1) nach einem der vorstehenden Ansprüche, **dadurch gekennzeichnet, dass** die Datenverarbeitungseinheit (16) dazu ausgebildet ist, zur Ermittlung der Druckamplitude (16) und der Druckbreite (18) aus dem Druckpuls (10) ein Frequenzanalyseverfahren, vorzugsweise mittels Fouriertransformation, auszuführen.

6.  Blutbehandlungsvorrichtung nach Anspruch 5, **dadurch gekennzeichnet, dass** als Referenzwert ein Druckpuls in Form eines Frequenzspektrums in der Datenverarbeitungseinheit (16) hinterlegt ist oder die Datenverarbeitungseinheit (16) dafür angepasst ist, einen in der Datenverarbeitungseinheit (16) hinterlegten Druckpuls mittels Frequenzanalyseverfahren in ein hinterlegtes Frequenzspektrum umzuwandeln, und die Datenverarbeitungseinheit (16) dafür angepasst ist, das Frequenzspektrum des erfassten Druckpulses (10) mit dem hinterlegten Frequenzspektrum zu vergleichen und vorzugsweise bei einer vorbestimmten minimalen Abweichung und/oder einer vorbestimmten maximalen Abweichung, insbesondere von zumindest einer Frequenz-Amplitude, besonders bevorzugt von einer Frequenz-Bereichs-Amplitude, den Zustand des Benachrichtigungssignals (18) zu ändern,

7.  Blutbehandlungsvorrichtung nach Anspruch 5, **dadurch gekennzeichnet, dass** als Referenzwert zumindest eine vorbestimmte Frequenz-Amplitude oder Frequenz-Bereichs-Amplitude hinterlegt ist, insbesondere eine Frequenz-Amplitude bei einem vordefinierten Füllstandsgrenzwert (22), und bei einem unterschreiten dieser hinterlegten Frequenz-Amplitude oder der Frequenz-Bereichs-Amplitude die Datenverarbeitungseinheit (16) den Zustand des Benachrichtigungssignals (18) ändert.

8.  Blutbehandlungsvorrichtung (1) nach einem der vorstehenden Ansprüche, **dadurch gekennzeichnet, dass**

    - der Füllstandsparameter (14) ein Maß für den Füllstand des Blutes (4) in dem Kammerbehältnis (6) darstellt und
    - die Datenverarbeitungseinheit (16) dazu ausgebildet ist, das Benachrichtigungssignal (18) in einen relevanten Zustand zu ändern, sobald der Füllstandsparameter (14) einen vordefinierten Füllstandsgrenzwert (22) unterschreitet.

9.  Blutbehandlungsvorrichtung (1) nach Anspruch 8, **dadurch gekennzeichnet, dass** die Datenverarbeitungseinheit (16) zusätzlich dazu ausgebildet ist, im Falle eines relevanten Zustandes des Benachrichtigungssignals (18) ein Ansteuersignal (24) zu erzeugen, welches die Blutpumpvorrichtung (8) so ansteuert, dass der Füllstand des Blutes (4) in dem Kammerbehältnis (6) erhöht wird, sobald der vordefinierte Füllstandsgrenzwert (22) unterschritten wird.

10. Blutbehandlungsvorrichtung (1) nach Anspruch 9, **dadurch gekennzeichnet, dass** die Datenverarbeitungseinheit (16) zusätzlich dazu ausgebildet ist, ein Alarmierungssignal (26) in einen relevanten Zustand zu ändern und zumindest die Alarmvorrichtung (20) zu aktivieren, sobald ein vordefinierter Häufigkeitsgrenzwert (28), der definiert wie häufig der Füllstand des Blutes (4) in dem Kammerbehältnis (6) in einem vordefinierten Zeitintervall den Füllstandsgrenzwert (22) unterschreitet, überschritten wird.

11. Blutbehandlungsvorrichtung (1) nach einem der vorstehenden Ansprüche, **dadurch gekennzeichnet, dass** der Druckbestimmungssensor (12) eine Abtastrate von zumindest 100Hz aufweist, um den zeitlichen Verlauf des Druckpulses (10) des Blutes (4) zu erfassen.

12. Blutbehandlungsvorrichtung (1) nach einem der vorstehenden Ansprüche, **dadurch gekennzeichnet, dass die** Blutpumpvorrichtung (8) zusätzlich eine Pegel- und/oder eine Level-Regulierungspumpe (42) aufweist und dass das Kammerbehältnis (6) zusätzlich eine ansteuerbare Belüftungsöffnung zum Belüften des Kammerbehältnisses (6) aufweist.

13. Verfahren zur Überwachung des Füllstandes des Blutes (4) in einer Blutbehandlungsvorrichtung (1) nach einem der Ansprüche 1 bis 12, umfassend die folgenden Schritte:

    - Erfassen des von der Blutpumpvorrichtung (8) eingeleiteten Druckpulses (10) durch den Druckbestimmungssensor (12);
    - Bestimmen einer Druckimpulsamplitude (36) und/oder einer Druckimpulsbreite (38) des erfassten Druckpulses (10) als Füllstandsparameter (14) durch die Datenverarbeitungseinheit (16),
    - Vergleichen der Füllstandsparameter (14) Druckimpulsbreite (38) und/oder Druckimpulsamplitude (36) mit zumindest einem in der Datenverarbeitungseinheit (16) hinterlegten Referenzwert durch die Datenverarbeitungseinheit (16);
    - Bestimmen einer Füllstandshöhe des Blutes (4) in dem Kammerbehältnis (6) anhand des Vergleiches mit dem Referenzwert durch die Datenverarbeitungseinheit (16);
    - kontinuierliches Analysieren und Verarbeiten de erfassten Füllstandsparameter (14) durch die Datenverarbeitungseinheit (16);
    - Verändern des Zustandes eines Benachrichtigungssignals (18) in Abhängigkeit des Füllstandsparameters (14) durch die Datenverarbeitungseinheit (16), sobald dieser einen vordefinierten Füllstandsgrenzwert (22) unterschreitet; und
    - Aktivieren der Alarmvorrichtung (20) durch die Datenverarbeitungseinheit (16) in Abhängigkeit des Zustandes des Benachrichtigungssignals (18).

14. Verfahren nach Anspruch 13, zusätzlich umfassend den folgenden Schritt:

    - kontinuierliches Ermitteln der Druckamplitude (16) und/oder der Druckbreite (18) aus dem erfassten Druckpuls (10) mittels eines Frequenzanalyseverfahren durch die Datenverarbeitungseinheit (16).

15. Verfahren nach einem der Ansprüche 13 und 14, zusätzlich umfassend die folgenden Schritte:

    - Verändern des Zustandes eines Alarmsignals (20) in einen relevanten Zustand durch die Datenverarbeitungseinheit (16), sobald der Füllstandsgrenzwert (22) einen vordefinierten Häufigkeitsgrenzwert (28) überschreitet und
    - Aktivierung der Alarmvorrichtung (20) durch die Datenverarbeitungseinheit (16) im Falle eines relevanten Zustandes des Alarmsignals (20).

**Claims**

1. A blood treatment device (1) for extracorporeal blood treatment, comprising a blood conducting system (2) with a chamber container (6) for separating bubbles from a blood (4) to be treated, and a blood pumping device (8) configured to pump the blood (4) and which in addition generates pressure pulses (10) with a predefined frequency in the blood conducting system (2),
   **characterized in that**
   the blood treatment device (1) further comprises:

    - a pressure detection sensor (12) in the blood conducting system (2) for detecting the pressure pulse (10) introduced by the blood pumping device (8),
    - a data processing unit (16) which is adapted to determine a pressure pulse amplitude (36) and/or a pressure pulse width (38) of the pressure pulse (10) from the detected pressure pulse (10), which serve as fill level parameter (14), to compare the fill level parameter (14) pressure pulse width (38) and/or pressure pulse amplitude (36) with at least one reference value stored in the data processing unit (16) to determine a fill level of the blood (4) in the chamber container (6) on the basis of the comparison with the reference value, and to change a state of a notification signal (18) as a function of the fill level parameters (14) and thus the fill level of the blood (4) in the chamber container (6),
    - at least one alarm device (20) which is activated as a function of the state of the notification signal (18).

2. The blood treatment device (1) according to claim 1, **characterized in that** the pressure pulse (10) detected after a first filling of the chamber container (6) with the blood (4) or a previously determined pressure pulse which represents a state of the chamber container (6) completely filled with blood (4) is stored in the data processing unit (16) as a reference value, and at a predetermined maximum deviation between the pressure pulse width (38) of the detected pressure pulse (10) and the pressure pulse width of the stored reference value and/or a predetermined maximum deviation between the pressure pulse amplitude (36) of the detected pressure pulse (10) and the pressure pulse amplitude of the reference value, the data processing unit (16) changes the state of the notification signal (18).

3. The blood treatment device (1) according to claim 1, **characterized in that** a detected pressure pulse (10) of the chamber container (6) at a predefined fill level threshold (22) is stored as reference value in the data processing unit (16) and, the data processing unit (16) changes the state of the notification signal (18) in the event of a predetermined minimum deviation between the pressure pulse width (38) of the detected pressure pulse (10) and the pressure pulse width of the stored reference value or an overshoot and/or a predetermined minimum deviation between the pressure pulse amplitude (36) of the detected pressure pulse (10) and the pressure pulse amplitude of the reference value or an undershoot.

4. The blood treatment device (1) according to one of the preceding claims, **characterized in that** the blood treatment device (1) comprises in its blood conducting system (2) exactly one pressure detection sensor (12) and with this one pressure detection sensor (12) is able to determine the fill level height of blood (4) in the chamber container (6).

5. The blood treatment device (1) according to one of the preceding claims, **characterized in that** the data processing unit (16) is adapted to perform a frequency analysis method, preferably via Fourier transformation, for determining the pressure amplitude (16) and the pressure width (18) from the pressure pulse (10).

6. The blood treatment device according to claim 5, **characterized in that** a pressure pulse in the form of a frequency spectrum is stored in the data processing unit (16) as a reference value or the data processing unit (16) is adapted to convert a pressure pulse stored in the data processing unit (16) into a stored frequency spectrum via a frequency analysis method, and the data processing unit (16) is adapted to compare the frequency spectrum of the detected pressure pulse (10) with the stored frequency spectrum and preferably to change the state of the notification signal (18) in the event of a predetermined minimum deviation and/or a predetermined maximum deviation, in particular of at least one frequency amplitude, particularly preferably of a frequency range amplitude.

7. The blood treatment device according to claim 5, **characterized in that** at least one predetermined frequency amplitude or frequency range amplitude is stored as a reference value, in particular a frequency amplitude at a predefined fill level threshold (22), and if this stored frequency amplitude or the frequency range amplitude is undershot, the data processing unit (16) changes the state of the notification signal (18).

8. The blood treatment device (1) according to one of the preceding claims, **characterized in that**

   - the fill level parameter (14) represents a measure for the fill level of the blood (4) in the chamber container (6), and
   - the data processing unit (16) is adapted to change the notification signal (18) to a relevant state as soon as the fill level parameter (14) falls below a predefined fill level threshold (22).

9. The blood treatment device (1) according to claim 8, **characterized in that** the data processing unit (16) is additionally adapted to generate a drive signal (24) in the event of a relevant state of the notification signal (18), wherein the drive signal (24) drives the blood pumping device (8) in such a way that the fill level of the blood (4) in the chamber container (6) is increased as soon as the predefined fill level threshold (22) is undershot.

10. The blood treatment device (1) according to claim 9, **characterized in that** the data processing unit (16) is additionally adapted to change an alarm signal (26) into a relevant state and to activate at least the alarm device (20) as soon as a predefined occurrence threshold (28), which defines how often the fill level of the blood (4) in the chamber container (6) falls below the fill level threshold (22) in a predefined time interval, is exceeded.

11. The blood treatment device (1) according to one of the preceding claims, **characterized in that** the pressure detection sensor (12) has a sampling rate of at least 100 Hz in order to detect the pressure pulse (10) of the blood (4) over time.

12. The blood treatment device (1) according to one of the preceding claims, **characterized in that** the blood pumping device (8) additionally comprises a gage and/or level regulation pump (42) and that the chamber container (6) additionally comprises a drivable ventilation opening for ventilating the chamber container (6).

13. A method for monitoring the fill level of blood (4) in a blood treatment device (1) according to one of claims 1 to 12, comprising the following steps:

- detecting the pressure pulse (10) introduced by the blood pumping device (8) by the pressure detection sensor (12);
- determining a pressure pulse amplitude (36) and/or a pressure pulse width (38) of the detected pressure pulse (10) as a fill level parameter (14) by the data processing unit (16),
- comparing the fill level parameters (14) pressure pulse width (38) and/or pressure pulse amplitude (36) with at least one reference value stored in the data processing unit (16) by the data processing unit (16);
- determining a fill level of the blood (4) in the chamber container (6) on the basis of the comparison with the reference value by the data processing unit (16);
- continuously analyzing and processing the detected fill level parameter (14) by the data processing unit (16);
- changing the state of a notification signal (18) in dependence of the fill level parameter (14) by the data processing unit (16) as soon as it falls below a predefined fill level threshold (22), and
- activating the alarm device (20) by the data processing unit (16) in dependence of the state of the notification signal (18).

14. The method according to claim 13, additionally comprising the following step:

- continuously determining the pressure amplitude (16) and/or the pressure width (18) from the detected pressure pulse (10) via a frequency analysis method by the data processing unit (16).

15. The method according to one of claims 13 and 14, additionally comprising the following steps:

- changing the state of an alarm signal (20) to a relevant state by the data processing unit (16) as soon as the fill level threshold (22) exceeds a predefined occurrence threshold (28), and
- activating the alarm device (20) by the data processing unit (16) in case of a relevant state of the alarm signal (20).

**Revendications**

1. Dispositif de traitement du sang (1) pour le traitement extracorporel du sang, présentant un système de conduite de sang (2) avec un récipient de chambre (6) pour la séparation de bulles d'un sang (4) à traiter, et un dispositif de pompage du sang (8) qui est conçu pour pomper le sang (4) et qui génère en outre des impulsions de pression (10) avec une fréquence prédéfinie dans le système de conduite de sang (2), **caractérisé en ce que** le dispositif de traitement du sang (1) présente en outre :

- un capteur de détermination de pression (12) dans le système de conduite de sang (2) pour détecter l'impulsion de pression (10) introduite par le dispositif de pompage de sang (8),
- une unité de traitement de données (16) qui est conçue pour déterminer, à partir de l'impulsion de pression (10) détectée, une amplitude d'impulsion de pression (36) et/ou une largeur d'impulsion de pression (38) de l'impulsion de pression (10), qui servent de paramètres de niveau de remplissage (14), pour comparer les paramètres de niveau de remplissage (14), la largeur d'impulsion de pression (38) et/ou l'amplitude d'impulsion de pression (36), à au moins une valeur de référence enregistrée dans l'unité de traitement de données (16), déterminer, à l'aide de la comparaison avec la valeur de référence, un niveau de remplissage du sang (4) dans le récipient de chambre (6) et modifier un état d'un signal de notification (18) en fonction des paramètres de niveau de remplissage (14) et donc du niveau de remplissage du sang (4) dans le récipient de chambre (6),
- au moins un dispositif d'alarme (20) qui est activé en fonction de l'état du signal de notification (18).

2. Dispositif de traitement du sang (1) selon la revendication 1, **caractérisé en ce que** l'impulsion de pression (10) détectée après un premier remplissage du récipient de chambre (6) avec le sang (4) ou une impulsion de pression déterminée au préalable, qui représente un état entièrement rempli de sang (4) du récipient de chambre (6), est enregistrée dans l'unité de traitement de données (16) comme valeur de référence, et, en cas d'écart maximal prédéterminé entre la largeur d'impulsion de pression (38) de l'impulsion de pression (10) détectée et la largeur d'impulsion de pression de la valeur de référence déposée et/ou d'écart maximal prédéterminé entre l'amplitude d'impulsion de pression (36) de l'impulsion de pression (10) détectée et l'amplitude d'impulsion de pression de la valeur de référence, l'unité de traitement de données (16) modifie l'état du signal de notification (18).

**3.** Dispositif de traitement du sang (1) selon la revendication 1, **caractérisé en ce que**, en tant que valeur de référence, une impulsion de pression détectée (10) du récipient de chambre (6) est enregistrée dans l'unité de traitement de données (16) en cas de valeur limite de niveau de remplissage prédéfinie (22) et l'unité de traitement de données (16) modifie l'état du signal de notification (18) en cas d'écart minimal prédéterminé entre la largeur d'impulsion de pression (38) de l'impulsion de pression (10) détectée et la largeur d'impulsion de pression de la valeur de référence mémorisée ou de dépassement vers le haut et/ou en cas d'écart minimal prédéterminé entre l'amplitude d'impulsion de pression (36) de l'impulsion de pression (10) détectée et l'amplitude d'impulsion de pression de la valeur de référence ou de dépassement vers le bas.

**4.** Dispositif de traitement du sang (1) selon l'une quelconque des revendications précédentes, **caractérisé en ce que** le dispositif de traitement du sang (1) présente exactement un capteur de détermination de pression (12) dans son système de conduite de sang (2) et est en mesure, avec ce capteur de détermination de pression (12), de déterminer le niveau de remplissage du sang (4) dans le récipient de chambre (6).

**5.** Dispositif de traitement du sang (1) selon l'une quelconque des revendications précédentes, **caractérisé en ce que** l'unité de traitement de données (16) est conçue pour exécuter un procédé d'analyse de fréquence, de préférence au moyen d'une transformation de Fourier, pour déterminer l'amplitude de pression (16) et la largeur de pression (18) à partir de l'impulsion de pression (10).

**6.** Dispositif de traitement du sang selon la revendication 5, **caractérisé en ce qu'**une impulsion de pression sous la forme d'un spectre de fréquence est enregistrée comme valeur de référence dans l'unité de traitement de données (16) ou **en ce que** l'unité de traitement de données (16) est adaptée pour convertir une impulsion de pression enregistrée dans l'unité de traitement de données (16) en un spectre de fréquence enregistré au moyen d'un procédé d'analyse de fréquence, et l'unité de traitement de données (16) est adaptée pour comparer le spectre de fréquence de l'impulsion de pression (10) détectée avec le spectre de fréquence enregistré et, de préférence, pour modifier l'état du signal de notification (18) en cas d'écart minimal prédéterminé et/ou d'écart maximal prédéterminé, en particulier d'au moins une amplitude de fréquence, le plus préférentiellement d'une amplitude de plage de fréquence,

**7.** Dispositif de traitement du sang selon la revendication 5, **caractérisé en ce qu'**au moins une amplitude de fréquence ou une amplitude de plage de fréquence prédéterminée est enregistrée comme valeur de référence, en particulier une amplitude de fréquence pour une valeur limite de niveau de remplissage (22) prédéfinie, et en cas de dépassement vers le bas de cette amplitude de fréquence enregistrée ou de l'amplitude de plage de fréquence, l'unité de traitement de données (16) modifie l'état du signal de notification (18).

**8.** Dispositif de traitement du sang (1) selon l'une quelconque des revendications précédentes, **caractérisé en ce que**

- le paramètre de niveau (14) représente une mesure du niveau de remplissage du sang (4) dans le récipient de chambre (6) et
- l'unité de traitement de données (16) est conçue pour modifier le signal de notification (18) en un état pertinent dès que le paramètre de niveau de remplissage (14) passe en dessous d'une valeur limite de niveau de remplissage (22) prédéfinie.

**9.** Dispositif de traitement du sang (1) selon la revendication 8, **caractérisé en ce que** l'unité de traitement de données (16) est en outre conçue pour générer, dans le cas d'un état pertinent du signal de notification (18), un signal de commande (24) qui commande le dispositif de pompage de sang (8) de sorte que le niveau de remplissage du sang (4) dans le récipient de chambre (6) est augmenté dès que la valeur limite de niveau de remplissage prédéfinie (22) n'est pas atteinte.

**10.** Dispositif de traitement du sang (1) selon la revendication 9, **caractérisé en ce que** l'unité de traitement de données (16) est en outre conçue pour modifier un signal d'alarme (26) en un état pertinent et pour activer au moins le dispositif d'alarme (20) dès qu'une valeur limite de fréquence (28) prédéfinie, qui définit à quelle fréquence le niveau de remplissage du sang (4) dans le récipient de chambre (6) passe en dessous de la valeur limite de niveau de remplissage (22) dans un intervalle de temps prédéfini, est dépassée.

**11.** Dispositif de traitement du sang (1) selon l'une quelconque des revendications précédentes, **caractérisé en ce que** le capteur de détermination de pression (12) présente une fréquence d'échantillonnage d'au moins 100 Hz afin de détecter l'évolution temporelle de l'impulsion de pression (10) du sang (4).

**12.** Dispositif de traitement du sang (1) selon l'une quelconque des revendications précédentes, **caractéri-**

sé en ce que le dispositif de pompage du sang (8) présente en outre une pompe de régulation de hauteur et/ou de niveau (42) et en ce que le récipient de chambre (6) présente en outre une ouverture d'aération pouvant être commandée pour aérer le récipient de chambre (6).

13. Procédé de surveillance du niveau de remplissage du sang (4) dans un dispositif de traitement du sang (1) selon l'une quelconque des revendications 1 à 12, comprenant les étapes suivantes consistant à :

> - détecter l'impulsion de pression (10) introduite par le dispositif de pompage de sang (8) au moyen du capteur de détermination de pression (12) ;
> - déterminer une amplitude d'impulsion de pression (36) et/ou une largeur d'impulsion de pression (38) de l'impulsion de pression détectée (10) en tant que paramètre de niveau de remplissage (14) au moyen de l'unité de traitement de données (16),
> - comparer les paramètres de niveau de remplissage (14), largeur d'impulsion de pression (38) et/ou amplitude d'impulsion de pression (36), à au moins une valeur de référence enregistrée dans l'unité de traitement de données (16) au moyen de l'unité de traitement de données (16) ;
> - déterminer un niveau de remplissage du sang (4) dans le récipient de chambre (6) en se basant sur la comparaison avec la valeur de référence au moyen de l'unité de traitement de données (16) ;
> - analyser et traiter en continu les paramètres de niveau de remplissage (14) détectés au moyen de l'unité de traitement de données (16) ;
> - modifier l'état d'un signal de notification (18) en fonction du paramètre de niveau (14) au moyen de l'unité de traitement de données (16) dès que celui-ci passe en dessous d'une valeur limite de niveau (22) prédéfinie ; et
> - activer le dispositif d'alarme (20) au moyen de l'unité de traitement de données (16) en fonction de l'état du signal de notification (18).

14. Procédé selon la revendication 13, comprenant en outre l'étape suivante consistant à :

> - déterminer en continu l'amplitude de pression (16) et/ou la largeur de pression (18) à partir de l'impulsion de pression (10) détectée via un procédé d'analyse de fréquence au moyen de l'unité de traitement de données (16).

15. 7Procédé selon l'une quelconque des revendications 13 et 14, comprenant en outre les étapes suivantes consistant à :

- changer l'état d'un signal d'alarme (20) en un état pertinent au moyen de l'unité de traitement de données (16) dès que la valeur limite de niveau (22) dépasse une valeur limite de fréquence (28) prédéfinie et
- activer le dispositif d'alarme (20) au moyen de l'unité de traitement de données (16) dans le cas d'un état pertinent du signal d'alarme (20).

Fig. 1

Fig. 2

Fig. 3a

Fig. 3b

Fig. 3c

Fig. 4a

Fig. 4b

Fig. 5

EP 3 877 017 B1

Fig. 6

EP 3 877 017 B1

**IN DER BESCHREIBUNG AUFGEFÜHRTE DOKUMENTE**

**In der Beschreibung aufgeführte Patentdokumente**

- WO 9301858 A
- US 9795731 B2
- WO 2006122737 A1
- US 2013049974 A1
- WO 2015188154 A9
- US 2016310657 A